# DEMANDE DE BREVET EUROPEEN

(11) **EP 4 477 153 A1**
(43) Date de publication de la demande: **18.12.2024**
(21) Numéro de dépôt: 24176316.8
(22) Date de dépôt: 16.05.2024
(51) Int. Cl.: A61B 8/08, A61B 5/00, G16H 50/30

(54) **PROCÉDÉ ET DISPOSITIF DE DÉTERMINATION DE L ÉTAT DU FOIE D'UN SUJET**

(30) Priorité: 16.06.2023 FR 2306223
(71) Demandeur: SUPERSONIC IMAGINE, 13290 Aix-en-Provence (FR)
(72) Inventeur: LAMBERT, William, 13008 Marseille (FR); ZHANG, Bo, 13109 Simiane-Collongue (FR); LAVAUD, Jonathan, 13090 Aix-en-Provence (FR)
(74) Mandataire: RVDB Nantes

(57) **Abrégé**

La présente divulgation concerne un procédé et un dispositif (10) de détermination de l'état du foie (3) d'un sujet (UR1). Le procédé comprend : obtention d'un premier groupe de données de scan (DT1a) à partir d'ondes ultrasonores (W) se propageant selon une première configuration de scan (CF1) du foie ; obtention d'un deuxième groupe de données de scan (DT1b) à partir d'ondes ultrasonores (W) se propageant selon une deuxième configuration de scan (CF2) d'un groupe de tissus du sujet ; et détermination, par une combinaison des premier et deuxième groupes de données de scan (DT1a, DT1b), d'un indicateur S3 représentatif de l'état du foie.

## Description

### Technique antérieure

La présente divulgation concerne la détermination de l'état du fois d'un sujet, tel qu'un humain ou un animal, et porte en particulier, mais pas exclusivement, sur l'aide au dépistage ou au diagnostic de troubles hépatiques tels que la stéatose hépatique non alcoolique (SHNA) (dite aussi NASH pour « non-alcoholic steatohepatitis »), dite aussi stéatopathie non alcoolique ou métabolique (NAFLD pour « Non Alcoholic Fatty Liver Disease »), ou encore la stéato-hépatite non alcoolique (NASH pour « non alcoholic steatohepatitis »).

La stéatose hépatique est un trouble du foie qui se caractérise par l'accumulation de graisse dans des cellules hépatiques. Il existe de multiples causes à la stéatose hépatique, dont en particulier l'alcool et le syndrome métabolique. La stéatose hépatique non alcoolique, dite SHNA, affectent les personnes qui ne boivent pas d'alcool de manière excessive. Lorsque plus de 5% des hépatocytes contiennent des gouttelettes lipidiques, on peut alors parler de stéatose hépatique pathologique. La stéatose hépatique simple, dite aussi stéatose, est généralement bénigne et ne cause donc pas de dommages importants au foie. En revanche, cette condition peut évoluer vers des troubles plus graves, tels que la stéato-hépatite non alcoolique, dite NASH, qui correspond à une affection agressive caractérisée par l'apparition d'une inflammation au sein du parenchyme hépatique. La stéatose-hépatite peut elle-même causer une accumulation de tissu cicatriciel (fibrose) pouvant éventuellement conduire à une maladie potentiellement grave du foie, à savoir la cirrhose.

La stéatose hépatique, en particulier la SHNA, est reconnue aujourd'hui comme un enjeu majeur de santé public à l'échelle mondiale. On recense actuellement environ deux millions de nouveaux cas de maladies du foie par an dans le monde. Une augmentation de 21% et 63% respectivement de la prévalence de la NAFLD et de la NASH entre 2015 et 2030 est envisagée à ce jour. Cette condition hépatique est liée à des facteurs comportementaux (activité physique, régime) et génétiques. Elle atteint notamment les personnes en surpoids ou obèses. Des études ont démontré que la stéatose hépatique non alcoolique fait augmenter la mortalité globale et la mortalité liée à la condition cardio-vasculaire, et peut conduire à diverses pathologies, notamment à des troubles hépatiques, à la formation de tumeurs, etc.

Cependant, la stéatose hépatique est un processus relativement lent et réversible, qui peut en particulier être traité efficacement notamment par une meilleure alimentation et de manière plus générale meilleure hygiène de vie, sous réserve toutefois d'une prise en charge précoce. Il est donc critique de pouvoir dépister et diagnostiquer au plus tôt les cas de stéatose hépatique, en particulier de NASH. Or, il existe à ce jour différentes techniques de diagnostic, et plus généralement de surveillance de l'état du foie, celles-ci n'offrant pas toujours des résultats satisfaisants.

L'examen par IRM est actuellement une des méthodes non invasives de référence pour évaluer l'état du foie, et en particulier pour diagnostiquer et quantifier la stéatose hépatique. Elle permet en particulier une inspection uniforme et non invasive sur la totalité du foie. Grâce à cette technique, il est ainsi possible de déterminer une valeur globale du taux de gras d'un foie, ce taux étant appelé PDFF pour « Proton Density Fat Fraction ». Cependant, cette technique implique certaines contraintes et limites, dans la mesure où elle nécessite des équipements et compétences encore peu disponibles (problème de coût, de complexité de fabrication, de rareté, de maintenance, de formation, etc.), voire peut nécessiter un paramétrage complexe ; ce qui la rend peu adaptée pour certains examens du foie, notamment pour des examens de routine, de surveillance de cas à grande échelle, et si possible très en amont dans le développement de potentielles maladies, afin de faire bénéficier aux sujets de traitements en amont, s'agissant de pathologies potentiellement souvent réversibles, sous réserve d'avoir été détectées suffisamment tôt.

La biopsie hépatique est également une technique de référence pour ce type de surveillance médicale et de diagnostic mais présente des risques et contraintes importants, en particulier en raison de son potentiel manque de fiabilité lié au caractère intrinsèquement très local de cet examen. Cette technique peut ainsi conduire à des erreurs de diagnostic, en particulier si la biopsie n'a pas ciblé le foie dans sa partie la plus impactée. A noter en outre qu'en raison de son caractère invasif, la biopsie hépatique peut conduire à des complications plus ou moins graves, tels que des saignements. Outre les problèmes de fiabilité et éventuels risques de complication, la biopsie présente un coût élevé sur le plan financier et en termes de temps investi par le sujet et le personnel soignant.

Les techniques ultrasonores ont été proposées pour le diagnostic non invasif de la stéatose hépatique, en particulier pour la SHNA à ses différents stades. Malgré son efficacité maintes fois démontrée, cette technique d'examen est toutefois parfois mal maîtrisée aujourd'hui et n'offre en conséquence pas toujours des résultats satisfaisants en termes notamment de pertinence des données acquises et traitées, de précision et de fiabilité. Une limite de cette technique réside en particulier dans la forte dépendance de la pertinence des résultats aux compétences du praticien échographe. Ceci s'explique notamment par le fait que cette technique se base sur une approche anatomique et qualitative, nécessitant la mise en oeuvre de nombreux paramètres, ce qui rend l'appréciation des résultats plus subjective et donc plus difficile car dépendant de l'utilisateur. Cette dépendance à l'utilisateur devient critique si ce dernier n'est pas suffisamment formé et/ou ne dispose pas de suffisamment de temps pour réaliser l'examen dans de bonnes conditions.

### Exposé de la divulgation

L'un des objets de la présente divulgation est de résoudre au moins l'un des problèmes ou déficiences décrits précédemment.

En particulier, il peut être souhaitable de réaliser une détermination (ou évaluation) précise et fiable de l'état du foie d'un sujet ou patient, tel qu'un sujet humain par exemple.

En particulier, il peut être souhaitable d'évaluer l'état du foie d'un sujet en vue de dépister précocement un disfonctionnement ou ses signes avant-coureurs ou diagnostiquer un trouble hépatique tel que la stéatose hépatique, en particulier la stéatose hépatique (ou stéatopathie) non alcoolique (SHNA), ou encore la stéato-hépatite non alcoolique (NASH).

A cet effet, selon un premier aspect, la présente divulgation vise un procédé de détermination de l'état du foie d'un sujet, ledit procédé comprenant :
- obtention d'un premier groupe de données de scan à partir d'ondes ultrasonores se propageant selon une première configuration de scan du foie ;
- obtention d'un deuxième groupe de données de scan à partir d'ondes ultrasonores se propageant selon une deuxième configuration de scan d'un groupe de tissus du sujet ; et
- détermination, par une combinaison des premier et deuxième groupes de données de scan, d'un indicateur S3 représentatif de l'état du foie.

Le procédé selon la divulgation peut comporter d'autres caractéristiques qui peuvent être prises séparément ou en combinaison, notamment parmi les modes de réalisation qui suivent qui sont présentés à titre d'illustration uniquement et peuvent être combinés ou associés sauf stipulation contraire.

Selon un exemple, le groupe de tissus du sujet correspond à une partie du sujet autre que le foie.

Selon un exemple, le procédé est tel que :
- la première configuration de scan est une configuration de scan intercostal du foie ; et
- la deuxième configuration de scan est une configuration de scan sous-costal du foie.

Selon un exemple, le procédé comprend :
- comparaison de l'indicateur S3 avec une valeur de référence.

Selon un exemple, le procédé comprend :
- évaluation d'un premier score (S1) à partir du premier groupe de données de scan ; et
- évaluation d'un deuxième score (S2) à partir du deuxième groupe de données de scan ;
dans lequel l'indicateur S3 est déterminé à partir des premier et deuxième scores.

Selon un exemple, le premier l'indicateur S1 et/ou le deuxième indicateur S2 comprennent au moins l'un parmi :
- un indicateur définissant un coefficient d'atténuation ;
- un indicateur définissant une vitesse du son ;
- un indicateur définissant un coefficient de rétrodiffusion ;
- un indicateur d'élasticité du foie ;
- un indicateur définissant une viscoélasticité du foie ;
- un indicateur de non-linéarité de propagation ;
- un indicateur de diffusion d'ondes ultrasonores ; et
- un indicateur de distribution de speckle ultrasonore.

Selon un exemple, le procédé est tel que :
- le premier score est un premier coefficient d'atténuation ; et
- le deuxième score est un deuxième coefficient d'atténuation ;
dans lequel l'indicateur S3 définit un biomarqueur d'un état physio-pathologique du foie.

Selon un exemple, l'un parmi les premier et deuxième groupes de données de scan est obtenu à partir d'un scan dit scan antérieur réalisé selon une configuration de scan antérieure et l'autre est obtenu à partir d'un scan dit scan postérieur réalisé selon une configuration de scan postérieure, le scan antérieur étant réalisé avant le scan postérieur ;
dans lequel la configuration de scan antérieure est l'une parmi les première et deuxième configuration de scan et la configuration de scan postérieure est l'autre parmi les première et deuxième configuration de scan.

Selon un exemple, la configuration de scan postérieure est sélectionnée à partir du premier ou deuxième score obtenu à partir du scan antérieur.

Selon un exemple, l'indicateur S3 est déterminé à partir d'une moyenne des premier et deuxième scores.

Selon un exemple, la moyenne est déterminée par une combinaison linéaire des premier et deuxième scores S1, S2.

Selon un exemple, la moyenne est une moyenne linéaire quadratique minimale.

Selon un exemple, la moyenne est déterminée par une combinaison non-linéaire des premier et deuxième scores S1, S2.

Selon un exemple, la détermination de l'indicateur S3 comprend :
- traitement des premier et deuxième groupes de données, ou de données obtenues à partir des premier et deuxième groupes de données, selon un modèle de traitement obtenu en utilisant un réseau de neurones entraîné à partir de données d'entraînement et de scores (ou indicateurs) de référence, les données d'entraînement comprenant des données de scan ultrasonores ou des données obtenues à partir des données de scan ultrasonores.

Selon un exemple, préalablement au traitement le réseau de neurones est entraîné par apprentissage machine au cours d'une phase d'entraînement comprenant :
- obtention des données de scan ultrasonores représentatives d'ondes se propageant selon les première et deuxième configurations de scan sur des sujets tests ;
- détermination des données d'entrainement à partir des données de scan ultrasonores ;
- obtention, à partir d'imageries IRM réalisés sur les sujets tests, des scores de référence représentatifs de l'état du foie des sujets tests ; et
- détermination, par comparaison des données d'entraînement avec les scores de référence, du modèle de traitement utilisé pour estimer l'indicateur S3 à partir de la combinaison des premier et deuxième groupes de données de scan.

Selon un exemple, le procédé comprend :
- évaluation, à partir de l'indicateur S3, d'un taux de masse grasse du foie du sujet.

Selon un deuxième aspect, la présente divulgation peut être mise en oeuvre par (ou impliquer) un programme d'ordinateur comprenant des instructions qui, lorsque le programme est exécuté par un ordinateur, participe à la mise en oeuvre du procédé selon le premier aspect. En particulier, les différentes étapes du procédé selon le premier aspect peuvent faire intervenir des instructions de programmes d'ordinateurs.

Un tel programme d'ordinateur peut utiliser n'importe quel langage de programmation ou équivalent, et il peut se trouver sous la forme d'un code source, d'un code objet, ou d'un code intermédiaire entre un code source et un code objet, tel que dans une forme partiellement compilée, ou sous n'importe quelle autre forme souhaitable.

Selon un troisième aspect, la présente divulgation concerne un support d'enregistrement (ou support d'informations), lisible par un ordinateur (ou un processeur), sur lequel est enregistré un programme d'ordinateur selon le deuxième aspect de la présente divulgation.

D'une part, le support d'enregistrement peut être n'importe quel entité ou dispositif capable de stocker le programme, telle qu'au moins une mémoire volatile et/ou non volatile. Par exemple, le support peut comporter un moyen de stockage, tel qu'une mémoire non-volatile réinscriptible, une mémoire ROM, un CD-ROM ou une mémoire ROM de type circuit microélectronique, ou encore un moyen d'enregistrement magnétique ou un disque dur. Cette mémoire peut par exemple comprendre une mémoire de carte graphique (ou carte vidéo), ce type de mémoire étant notamment apte à traiter des données d'image (ou données vidéo).

D'autre part, ce support d'enregistrement peut également être un support transmissible tel qu'un signal électrique ou optique, un tel signal pouvant être acheminé via un câble électrique ou optique, par radio classique ou hertzienne ou par faisceau laser autodirigé ou par d'autres moyens. Le programme d'ordinateur selon la présente divulgation peut être en particulier téléchargé grâce à un réseau filaire ou non, de type local ou non (Bluetooth^{®} par exemple, Wi-Fi, Ethernet, Internet, 4G, 5G ou autres).

Alternativement, le support d'enregistrement peut être un circuit intégré dans lequel le programme d'ordinateur est incorporé, le circuit intégré étant adapté pour exécuter ou pour être utilisé dans l'exécution du procédé en question.

Selon un quatrième aspect, la présente divulgation concerne un dispositif de détermination (ou dispositif de surveillance) configuré pour déterminer l'état du foie d'un sujet en mettant en oeuvre le procédé de détermination du premier aspect de la présente divulgation. A cet effet, le dispositif de détermination peut comprendre des modules configurés pour mettre en oeuvre respectivement les différentes étapes du procédé du premier aspect de la divulgation.

Selon un exemple, le dispositif de détermination comprend une mémoire associée à un processeur, cette mémoire comprenant un programme d'ordinateur selon le deuxième aspect de la divulgation.

Selon un exemple, le dispositif de détermination de l'état du foie d'un sujet, ce dispositif comprenant :
- un premier module d'obtention configuré pour obtenir un premier groupe de données de scan à partir d'ondes ultrasonores se propageant selon une première configuration de scan du foie ;
- un deuxième module d'obtention configuré pour obtenir un deuxième groupe de données de scan à partir d'ondes ultrasonores se propageant selon une deuxième configuration de scan d'un groupe de tissus du sujet ; et
- un module d'analyse configuré pour déterminer, par une combinaison des premier et deuxième groupes de données de scan, indicateur S3 représentatif de l'état du foie.

Selon un mode de réalisation, l'invention est mise en oeuvre au moyen de composants logiciels et/ou matériels. Le dispositif de détermination peut en particulier comprendre des modules configurés pour mettre en oeuvre les étapes du procédé selon le premier aspect de la divulgation. Ainsi, les différents modes de réalisation mentionnés dans ce document en relation avec le procédé selon le premier aspect de la divulgation ainsi que les avantages associés peuvent s'appliquer de façon analogue au dispositif de détermination selon le quatrième aspect de la présente divulgation.

Dans cette optique, le terme « module » peut correspondre dans la présente divulgation aussi bien à un composant logiciel, qu'à un composant matériel ou à un ensemble de composants matériels et logiciels. Un composant logiciel correspond à un ou plusieurs programmes d'ordinateur, un ou plusieurs sous-programmes d'un programme, ou de manière plus générale à tout élément d'un programme ou d'un logiciel apte à mettre en oeuvre une fonction ou un ensemble de fonctions, selon ce qui est décrit ci-dessous pour le module concerné.

De la même manière, un composant matériel correspond à tout élément d'un ensemble matériel (ou hardware) apte à mettre en oeuvre une fonction ou un ensemble de fonctions, selon ce qui est décrit ci-dessous pour le module concerné.

Selon un cinquième aspect, la présente divulgation concerne un système de détermination de l'état du foie d'un sujet, ce système comprenant :
- un dispositif de détermination selon le quatrième aspect de la présente divulgation ; et
- un système à ultrasons couplé au dispositif de détermination.

Le procédé et le dispositif de détermination de la présente divulgation permettent avantageusement de déterminer de façon fiable, reproductible et performante l'état du foie d'un sujet, qui peut être sain ou présenter un état physio-pathologique quelconque du foie. Pour ce faire, la présente divulgation s'appuie sur la combinaison de données de scan (ou données ultrasonores) obtenues selon deux configurations de scan distinctes, à savoir selon respectivement une première configuration de scan CF1 visant le foie et une deuxième configuration de scan CF2 visant le foie ou un autre groupe de tissus du sujet. La combinaison de ces données de scan permet avantageusement d'obtenir un indicateur S3 représentant de façon plus fiable et robuste l'état du foie du sujet considéré que si une seule configuration de scan était utilisée.

Le procédé et le dispositif de détermination impliquent donc l'obtention de données de scan selon des configuration de scan différentes. Un scan au sens de la présente divulgation correspond à un scan échographique (ou ultrasonores), une observation échographique (ou ultrasonore) ou encore une phase d'observation par échographie (ou ultrasons). Un tel scan comprend la propagation d'ondes ultrasonores dans un milieu, à savoir le sujet examiné, et la récupération en retour d'échos échographiques (ou échos ultrasonores) représentatifs de l'état du foie du sujet. Chaque scan permet d'obtenir (ou générer) des données de scan représentatives d'une ou plusieurs régions cibles (ou zones d'intérêt) du sujet.

La ou les régions cibles du sujet qui sont examinées (ou scannées) par ultrasons au cours d'un scan échographique sont fonction de la configuration de scan utilisée. Divers paramètres peuvent définir cette configurations, dont notamment l'orientation et/ou la position du dispositif émetteur lors du scan échographique.

La combinaison de deux modes d'acquisition distincts, par exemple mode intercostal et mode sous-costal, permet avantageusement d'améliorer la pertinence des données d'acquisition par rapport à une technique conventionnelle où seules des données ultrasonores obtenues en mode intercostal seraient prises en compte. Les données ultrasonores ainsi acquises sont plus représentatives de la condition du foie. Le recours à deux configurations de scan distinctes permet avantageusement de diversifier les mesures et ainsi augmenter la fiabilité et la précision des résultats.

En outre, dans le cas de la stéatose hépatique par exemple, toutes les régions du foie du sujet peuvent ne pas être affectées de la même manière. Certains cas de stéatoses hétérogènes ont ainsi été constatés. La multiplication des scans ultrasonores selon des configurations de scan différentes permet d'obtenir une estimation plus représentative de l'état global du foie du sujet.

La présente divulgation peut ainsi avantageusement fournir une aide au diagnostic ou à la surveillance de certains états physio-pathologiques du foie, tels que la stéatose hépatiques (par exemple de type SHNA), la stéatose inflammatoire, etc.

Les caractéristiques et avantages de la divulgation apparaitront plus précisément à la lecture de la description qui va suivre, donnée uniquement à titre d'exemple non limitatif, et faite en référence aux figures annexées. Notamment, les exemples illustrés dans les figures peuvent être combinés entre eux, sauf incohérence mentionnée ou notoire.

### Brève description des figures

D'autres caractéristiques et avantages de la présente divulgation ressortiront de la description des exemples de réalisation non limitatifs de la présente divulgation ci-après, en référence aux figures 1 à 16 annexées, sur lesquelles :
[Fig. 1] illustre schématiquement une configuration de scan intercostale d'un scan ultrasonore du foie du sujet, selon un exemple particulier ;
[Fig. 2] est une image échographique illustrant l'apparition de zones d'ombres lors de l'examen échographique du foie d'un sujet en mode d'acquisition intercostal ;
[Fig. 3] représente schématiquement un dispositif de détermination et un système à ultrasons, selon un exemple de réalisation de la présente divulgation ;
[Fig. 4] représente schématiquement un système à ultrasons, selon un exemple de réalisation de la présente divulgation ;
[Fig. 5] représente schématiquement un dispositif de détermination, selon un exemple de réalisation de la présente divulgation ;
[Fig. 6] représente schématiquement des configurations de scan selon lesquelles sont réalisés des scans ultrasonores, selon un exemple de réalisation de la présente divulgation ;
[Fig. 7] représente schématiquement des configurations de scan selon lesquelles sont réalisés des scans ultrasonores, selon un exemple de réalisation de la présente divulgation ;
[Fig. 8] représente schématiquement un procédé de détermination réalisé par le dispositif de détermination de la figure 3, selon un exemple de réalisation de la présente divulgation ;
[Fig. 9] représente schématiquement des résultats obtenus en mode d'acquisition intercostal, selon un exemple de réalisation de la présente divulgation ;
[Fig. 10] représente schématiquement des résultats obtenus en mode d'acquisition sous-costale, selon un exemple de réalisation de la présente divulgation ;
[Fig. 11] représente schématiquement des résultats obtenus selon le procédé de détermination en combinant les résultats des figures 9 et 10, selon un exemple de réalisation de la présente divulgation ;
[Fig. 12] représente schématiquement la distribution des scores PDFF du foie de sujets tests lors d'une étude validant l'efficacité du procédé de détermination, selon un exemple de réalisation de la présente divulgation ;
[Fig. 13] représente schématiquement un procédé de détermination, selon un exemple de réalisation de la présente divulgation ;
[Fig. 14] représente schématiquement un procédé de détermination, selon un exemple de réalisation de la présente divulgation ;
[Fig. 15] représente schématiquement un réseau de neurones mis en oeuvre par le dispositif de détermination de la figure 3, selon un exemple de réalisation de la présente divulgation ; et
[Fig. 16] représente schématiquement une phase d'entraînement réalisé par le réseau de neurones de la figure 15, selon un exemple de réalisation de la présente divulgation.

### Description des modes de réalisation

La présente divulgation porte notamment sur des dispositifs et procédés de détermination de l'état du foie d'un sujet, à des fins par exemple de campagne de dépistage, suivi médical ou d'aide au diagnostic.

Il est connu de réaliser l'examen du foie d'un sujet par échographie, notamment en vue de dépister ou diagnostiquer des troubles hépatiques tels que la stéatose hépatique (de type NAFLD par exemple) ou encore la stéato-hépatite de type NASH. Le recours à cette technique est avantageux en particulier en raison de son caractère non invasif, de la relative disponibilité sur le terrain des appareils échographiques pour les examens médicaux, de la facilité de les déplacer selon les besoins, et des coûts et risques des investissements et des examens moins élevés par rapport par exemple à l'IRM et la biopsie.

Toutefois, cette technique d'examen, dont la pertinence des résultats est conditionnée grandement par le niveau de qualification du praticien, est encore à ses débuts et n'offre pas toujours des résultats satisfaisants, notamment en termes de performances et de fiabilité. Ces problèmes peuvent avoir plusieurs origines comme discuté ci-après.

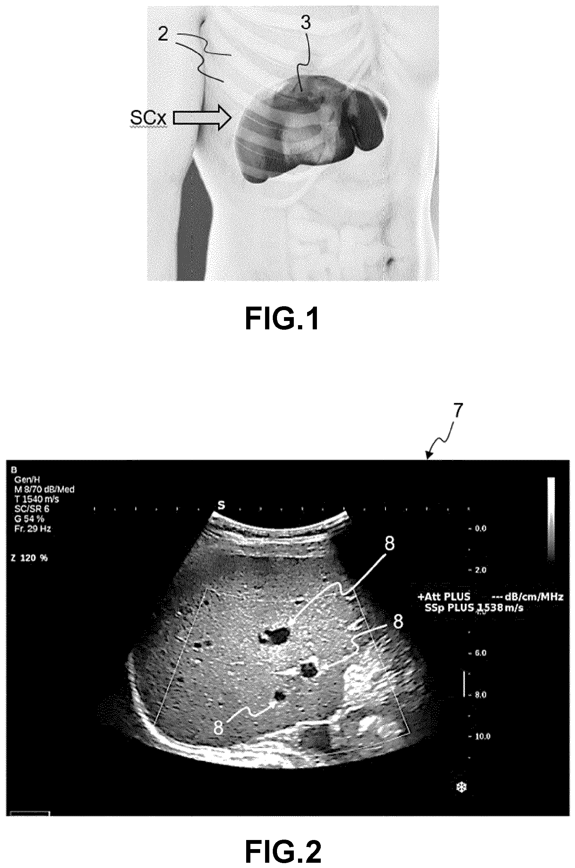

Comme illustré en figure 1, la pratique largement établie à l'échelle mondiale consiste actuellement à réaliser un tel examen échographique du foie selon une configuration de scan dite « intercostale », notée SCx (figure 1), c'est-à-dire en positionnant la sonde ultrasonore de sorte que les ondes ultrasonores se propagent entre les côtes 2 du sujet afin de récupérer en retour un écho ultrasonore représentatif du foie 3 du sujet. Pour ce faire, on positionne la sonde à ultrasons, éventuellement enduite de gel approprié, en contact avec la peau du sujet au niveau de sa cage thoracique, de sorte à scanner « au travers des côtes », c'est-à-dire entre les côtes.

Cette configuration de scan intercostale est considérée comme la plus fiable, notamment en raison du fait que les côtes du sujet protègent les tissus sous-jacents et permettent donc de limiter les déformations que peut causer la sonde lorsqu'elle est appliquée en contact sur le patient. De telles déformations risquent en effet de fausser les résultats de l'observation, les tissus comprimés se comportant alors différemment. La configuration intercostale est également privilégiée dans la mesure où cela facilite l'examen, en particulier pour certains sujets difficiles à examiner (notamment ceux présentant un haut niveau de gras sous-cutané). Le manipulateur échographiste peut prendre pour repère les côtes du sujet pour orienter et positionner au mieux la sonde du système d'échographie.

Pour notamment les raisons mentionnées ci-dessus, les études cliniques recommandent actuellement d'adopter la configuration de scan intercostale pour réaliser un examen du foie, en particulier en vue de dépister ou diagnostiquer des troubles hépatiques tels que ceux déjà évoquées.

Il a cependant été constaté que l'examen échographique du foie selon la technique intercostale pose certains problèmes et impliquent des limites qu'il convient d'adresser. Comme illustré à titre d'exemple en figure 2, il a notamment été observé l'apparition de zones d'ombres 8 dans les images échographiques 7 obtenues selon la configuration de scan intercostale. Ces zones obscures 8, qui gênent l'observation, s'expliquent par la forme généralement convexe de la sonde à ultrasons qui ne permet pas toujours à cette dernière d'être totalement en contact avec la peau du sujet lors de l'examen, en particulier en raison des côtes du sujet qui forment des reliefs peu compatibles avec la forme de la sonde. Les zones de non-contact entre la sonde et la peau du sujet au cours de l'observation échographique peuvent gêner la propagation des ondes ultrasonores et conduire à la formation de ces zones qui apparaissent alors comme obscures 8 à l'image.

Il est toutefois difficile voire non souhaitable de modifier la forme de la sonde pour mieux épouser le profil des côtes, notamment car les configurations actuelles sont conçues pour maximiser le champ de vue du système échographique et assurent une certaine polyvalence de la sonde, ce qui permet à cette dernière d'être adaptée pour réaliser divers examens échographiques.

Outre les problèmes de zones d'ombre mentionnées ci-dessus, l'observation échographique du foie selon la configuration classique de scan intercostale souffre de manière générale de résultats parfois insatisfaisants. Il a été constaté que l'évaluation de certaines caractéristiques du foie, tels que certains biomarqueurs, n'est pas toujours suffisamment performante ou fiable dans le cas de telles utilisations.

La présente divulgation se propose donc de fournir une solution notamment aux problèmes et déficiences mentionnés ci-avant, permettant de déterminer avec plus de fiabilité et de meilleures performances l'état du foie d'un sujet, tel qu'un être humain ou un animal, ou plus généralement l'état du foie d'un être vivant par exemple, notamment mais pas exclusivement pour aider au dépistage ou au diagnostic de troubles hépatiques, ou plus généralement pour évaluer l'état du foie du sujet. Cette solution s'appuie en particulier sur un procédé et un dispositif de détermination de l'état du foie d'un sujet.

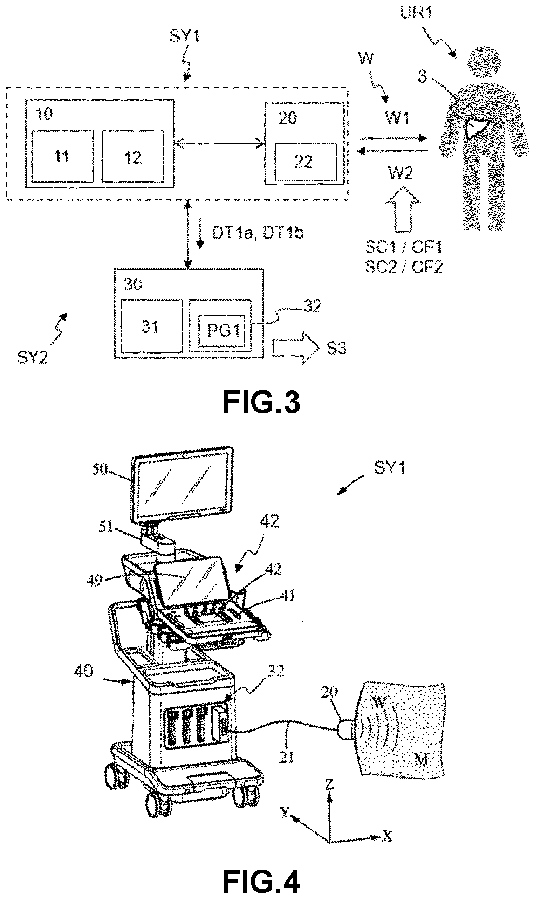

Des procédés de détermination et dispositifs de détermination (dits aussi « procédés » et « dispositifs ») vont être décrits dans ce qui va suivre selon des modes particuliers de réalisation de la divulgation en référence conjointement aux figures 3 à 16. Sauf indications contraires, les éléments communs ou analogues à plusieurs figures portent les mêmes signes de référence et présentent des caractéristiques identiques ou analogues, de sorte que ces éléments communs ne sont généralement pas à nouveau décrits par souci de concision.

Les termes « premier(s) » (ou première(s)), « deuxième(s) », etc.) sont utilisés dans ce document par convention arbitraire pour permettre d'identifier et de distinguer différents éléments (tels que des opérations, des dispositifs, etc.) mis en oeuvre dans les modes de réalisation décrits ci-après.

Comme illustré en figure 3, on décrit par la suite à titre d'exemple des modes de réalisation de la présente divulgation dans lesquels le foie 3 d'un sujet UR1 est observé par échographique au moyen d'un système à ultrasons noté SY1. Pour ce faire, des ondes ultrasonores W sont émises dans différentes régions (ou zones d'intérêt) du sujet UR1 au moyen du système ultrasonore SY1 afin d'obtenir des données ultrasonores représentatives de l'état du foie 3. Cette observation comprend plusieurs scans échographiques, c'est-à-dire plusieurs phases au cours desquels se propagent des ondes ultrasonores et sont récupérées en retour des échos ultrasonores représentatifs de l'état du foie 3. Autrement dit, au cours de chaque scan, une ou des régions du foie sont insonifiées par des ondes ultrasonores incidentes et des échos rétrodiffusés par ces régions du foie sont captés en retour au moyen d'une sonde ultrasonore. On utilisera par la suite de façon interchangeable les termes « scan », « scan échographique (ou ultrasonores) », « observation échographique (ou ultrasonore) », « phase d'observation par échographie (ou ultrasons) » ou encore « phase d'acquisition ».

Dans les exemples considérés, le sujet est un être vivant, à savoir un mammifère, par exemple un être humain. A noter toutefois que le concept de la présente divulgation peut s'appliquer plus généralement à un quelconque sujet pourvu d'un foie, (humain ou animal), vivant ou mort, dont on souhaite évaluer l'état du foie.

A noter que le sujet UR1 utilisé pour illustrer la présente divulgation peut avoir un corps se trouvant dans un état physiologique quelconque, notamment au niveau hépatique. Le foie 3 du sujet peut en particulier être sain (par exemple aucun trouble hépatique particulier) ou souffrir d'un quelconque trouble ou pathologie hépatique.

Les procédé et dispositif de la présente divulgation s'appuient notamment sur l'obtention de deux groupes de données, à partir respectivement d'ondes ultrasonores W se propageant au cours de scans ultrasonores, d'un même sujet, selon des configurations de scan différentes, au moins l'une de ces configurations étant une configuration de scan du foie, et sur la combinaison de ces deux groupes de données pour déterminer un indicateur noté S3 (dit aussi indicateur global, ou score global, ou paramètre) qui est représentatif de l'état du foie 3 du sujet UR1. La combinaison de ces deux groupes de données permet avantageusement de déterminer un indicateur représentatif de l'état du foie du sujet.

Une configuration de scan du foie désigne dans la présente divulgation une configuration de scan selon laquelle des données échographiques représentatives du foie du sujet sont obtenues. Autrement dit, un scan échographique réalisé selon une configuration de scan du foie scrute (ou vise, ou cible), au moins en partie, le foie du sujet, de sorte à obtenir des données échographiques représentatives dudit foie.

Selon des modes particuliers de réalisation, le procédé de détermination comprend :
- obtention d'un premier groupe de données de scan à partir d'ondes ultrasonores se propageant selon une première configuration de scan du foie ;
- obtention d'un deuxième groupe de données de scan à partir d'ondes ultrasonores se propageant selon une deuxième configuration de scan d'un groupe de tissus du sujet ; et
- détermination, par une combinaison des premier et deuxième groupes de données de scan, d'un indicateur S3 représentatif de l'état du foie

Comme illustré à titre d'exemple en figure 3, on considère par la suite différents scans (ou observations) échographiques, à savoir un premier scan SC1 et un deuxième scan SC2, qui sont réalisés au moyen du système à ultrasons SY1 sur un sujet UR1 selon des configurations (ou modes) de scan différentes - notées respectivement CF1 et CF2 - en vue de déterminer l'état du foie 3 du sujet UR1. Les configurations de scan CF1 et CF2 peuvent varier selon le cas, en termes notamment de direction de scan et/ou de position par rapport au sujet, dans la mesure toutefois où ces configurations sont légèrement ou plus globalement différentes l'une de l'autre.

Un dispositif de détermination (dit aussi dispositif de traitement) 30 est en outre configuré pour obtenir et traiter des données de scan (ou données ultrasonores) DT1a et DT1b générées par le système à ultrasons SY1 à partir respectivement des premier et deuxième scans SC1 et SC2 (figure 3). Pour ce faire, le dispositif de détermination 30 coopère (est couplé) avec le système à ultrasons SY1 pour mettre en oeuvre le procédé de détermination selon au moins un mode particulier de réalisation.

Comme illustré en figure 3, le dispositif de détermination 30 et le système à ultrasons SY1 forment ensemble un système de détermination (ou système d'analyse) SY2, ce système étant configuré pour déterminer l'état du foie d'un sujet.

Selon un exemple, le dispositif de détermination 30 fait partie intégrante du système à ultrasons SY1. Selon une variante, le dispositif de détermination 30 est externe au (distinct du) système à ultrasons SY1.

Le système SY1 peut être un système médical à ultrasons. En conséquence, le dispositif émetteur 20 peut être une (ou faire partie, au moins partiellement, d'une) sonde médicale et/ou à ultrasons.

Des exemples de réalisation du système à ultrasons SY1 sont à présent décrits à titre purement illustratif.

La figure 3 représente schématiquement un exemple de réalisation du système à ultrasons SY1 qui comprend un dispositif de contrôle (ou dispositif de pilotage) 10 et un dispositif émetteur 20. Le dispositif de contrôle 10 est configuré pour piloter le dispositif émetteur 20. Le dispositif émetteur 20 est configuré pour émettre et recevoir des ondes ultrasonores W, vers et depuis un milieu d'étude, à savoir le sujet UR1 dans les exemples considérés, et ce sous le contrôle du dispositif de contrôle 10.

Selon un exemple particulier, tout ou partie du dispositif de détermination 30 fait partie du dispositif de contrôle 10. Selon un exemple, les dispositifs 10 et 30 forment un seul et même dispositif.

Le dispositif émetteur 20 est par exemple un dispositif transducteur à ultrasons (ou sonde à ultrasons) configuré pour émettre et/ou recevoir des ondes ultrasonores W. Ce dispositif émetteur 20 est par exemple compris dans une sonde ultrasonore destinées à être appliquée en contact avec le sujet UR1 au cours de scans échographiques pour déterminer l'état de son foie 3.

A noter que diverses configurations du dispositif émetteur 20 sont toutefois possibles. En particulier, tout ou partie du dispositif de contrôle 10 peut être mis en oeuvre dans le dispositif émetteur 20 (par exemple dans la sonde). Tout ou partie du dispositif de contrôle 10 peut être externe au dispositif émetteur 20 (ou à la sonde).

Le système de contrôle 10 peut comprendre dans cet exemple une unité (ou module) de traitement 11 et une unité (ou module) de commande 12. L'unité de commande 12 (dite aussi unité de commande de puissance) est configurée pour piloter le dispositif émetteur 20 au moyen de signaux électriques qui sont échangés (ou transmis) entre le système 10 et le dispositif transducteur 20. L'unité de commande 12 génère ainsi des signaux électriques qui sont transmis au dispositif transducteur 20 pour causer l'émission, par ledit dispositif transducteur 20, d'ondes ultrasonores W1 en direction de la zone d'intérêt, située dans le sujet UR1. Les signaux électriques ainsi générés sont représentatifs des (ou définissent les) ondes ultrasonores projetées dans le milieu. À cette fin, l'unité de commande 12 peut par exemple être ou comprendre au moins un pulseur électronique, dit aussi « pulseur ».

L'unité de commande 12 peut éventuellement comprendre des dispositifs récepteurs ou circuits récepteurs (non représentés) configurés pour recevoir des signaux électriques en provenance du dispositif transducteur 20.

L'unité de traitement 11 peut être configurée pour commander l'unité de commande 12, par exemple en commandant les signaux électriques. Cette unité de traitement 11 peut par exemple être ou comprendre au moins un processeur.

Selon un exemple, l'unité de traitement 11 et/ou l'unité de pilotage 12 sont comprises dans le corps 31 (élément central) du système SY1 représenté à titre illustratif en figure 4.

Plus particulièrement, l'unité de traitement 11 peut être configurée pour contrôler des signaux électriques qui sont générés par l'unité de commande 12. L'unité de traitement 11 peut également être configurée pour traiter (et/ou interpréter) des signaux électriques éventuellement reçus par l'unité de commande 12 en provenance du dispositif émetteur 20. Ces signaux sont représentatifs d'ondes W2 reçues par le dispositif transducteur 20 en provenance du sujet UR1 (figure 3). Ces ondes W2 forment par exemple un ou plusieurs échos ultrasonores, c'est-à-dire une réponse du milieu étudié (à savoir le foie du sujet UR1) aux ondes ultrasonores émises en direction dudit milieu. Le traitement réalisé par l'unité de traitement 11 sur les signaux reçus peut varier selon le cas et comprendre par exemple au moins l'une quelconque parmi des opérations d'amplification, de filtrage, de numérisation et de conditionnement des signaux.

Le terme « milieu » désigne dans cette divulgation une ou des zones d'intérêt (ou régions) du sujet UR1, c'est-à-dire les zones qui sont insonifiées ou scrutées par ultrasons, à savoir des zones d'intérêt du foie 3 et éventuellement des zones d'intérêt correspondant à d'autres parties du sujet UR1.

Comme illustré en figure 3, le dispositif émetteur 20 peut comprendre par exemple un ou plusieurs transducteurs ultrasonores (dit aussi éléments transducteurs) 22, chacun étant configuré pour convertir un signal électrique reçu du dispositif de contrôle 10 en ondes ultrasonores (et éventuellement aussi réciproquement). Les transducteurs 22 peuvent ainsi être configurés pour émettre des ondes (ou impulsions ultrasonores, ou faisceaux ultrasonores) W1 dans le milieu, ce qui correspond à une opération d'émission.

Les transducteurs 22 peuvent éventuellement aussi être configurés pour recevoir lors d'une opération de réception des signaux ultrasonores W2 en provenance d'une ou plusieurs zones d'intérêt du sujet UR1, par exemple en réponse aux ondes W1 transmises. Ces signaux W2 reçus peuvent prendre la forme d'un ensemble de signaux d'écho rétrodiffusés en réponse aux signaux W1 préalablement émis. Chaque transducteur 22 peut par exemple convertir un signal d'écho W2 reçu en un signal électrique. Le signal peut ensuite être traité par le dispositif de contrôle 10 (par exemple par l'unité de traitement 11) ou par tout système associé (dédié), directement connecté ou non. Par exemple, le signal W2 peut être amplifié, filtré, numérisé et/ou une opération de conditionnement du signal peut être effectuée. Les éléments transducteurs peuvent être disposés selon une ligne de transducteurs, une matrice, ou comme un réseau de transducteurs ou toute autre configuration.

A noter que le ou les mêmes transducteurs 22 peuvent être utilisés pour émettre des ondes (ou impulsions) W1 et, le cas échéant, recevoir les ondes W2 formant la réponse du milieu, ou des transducteurs différents peuvent être utilisés pour l'émission et la réception des ondes. Il peut y avoir un ou plusieurs transducteurs d'émission, et éventuellement une pluralité de transducteurs de réception. Dans une autre variante, un même transducteur 22 peut être utilisé pour l'émission et la réception des ondes ultrasonores. Les transducteurs 22 peuvent comprendre des cristaux piézoélectriques et/ou d'autres composants qui peuvent être configurés pour générer et/ou enregistrer et/ou recevoir des signaux.

Le dispositif de contrôle 10 peut comprendre le dispositif émetteur 20. En variante, le dispositif émetteur 20 peut être externe au dispositif de contrôle 10. Par exemple, le dispositif émetteur 20 peut être connecté au dispositif de contrôle 10 par un câble ou peut communiquer sans fil avec lui. Dans ce dernier cas, le dispositif émetteur 10 peut par exemple comprendre une batterie et recevoir des signaux de communication du dispositif de contrôle 10 qui représentent les signaux électriques (par exemple les fréquences de pilotage et/ou toute information comprise dans les signaux électriques). Le dispositif émetteur 20 peut alors reproduire les signaux électriques en interne à partir des signaux de communication reçus.

Le dispositif émetteur 20 peut par exemple être un dispositif émetteur d'ultrasons conventionnel. Ainsi, une différence selon la présente divulgation peut résider dans la manière dont les données ultrasonores DT1a et DT1b sont obtenues et traitées par le dispositif de détermination 30 pour déterminer l'état du foie 3 du sujet UR1, comme décrit plus en détail ci-après.

Le dispositif de contrôle 10 peut être stationnaire ou mobile. Le dispositif émetteur 20 peut également être stationnaire ou mobile. Par exemple, le dispositif de contrôle 10 peut être un système fixe (par exemple comprenant une unité de traitement et un dispositif d'affichage, comme décrit ci-dessous) et le dispositif émetteur 20 peut être mobile (par exemple un dispositif capteur, un dispositif de mesure, ou une sonde). Toutefois, il est également possible que le dispositif émetteur 20 soit intégré au dispositif de contrôle 10, et que le dispositif 10 soit un système mobile. Par exemple, le dispositif de contrôle 10 peut être configuré pour être piloté de manière autonome, par exemple grâce à une batterie incluse. D'autres exemples sont décrits ci-dessous.

Selon un exemple, le dispositif de contrôle 10 peut comprendre au moins une mémoire (non représentée) utilisée par l'unité de traitement 11 pour commander l'unité de commande 12. Cette mémoire peut éventuellement faire partie de l'unité de traitement 11. Dans des exemples, le processeur et la mémoire de l'unité de traitement 11 peuvent être incorporés dans le dispositif de contrôle 10 illustré en figure 3 ou peuvent être incorporés dans un ordinateur ou un dispositif informatique lié de manière communicante à celui-ci.

Selon la configuration et le type de dispositif informatique considéré, la mémoire du dispositif de contrôle 10 peut être volatile (telle que la RAM), non volatile (telle que de la mémoire ROM, flash, EEPROM, etc. ou tout autre dispositif de stockage et/ou support lisible par ordinateur comme décrit ci-après) ou une combinaison des deux. Cette mémoire peut, par exemple, être gérée en mode DMA (pour « Direct Memory Access » en anglais). La mémoire utilisée par l'unité de traitement 11 peut par exemple comprendre tout ou partie d'une mémoire de carte graphique (ou carte vidéo), ce type de mémoire étant notamment apte à traiter et/ou envoyer des données d'image pouvant être utilisées pour afficher une ou des images sur un écran (ou une unité) d'affichage.

Plus généralement, le dispositif de contrôle 10 peut comprendre des dispositifs de stockage (amovibles et/ou non amovibles), y compris, mais sans s'y limiter, des disques magnétiques ou optiques ou des bandes.

En outre, le dispositif de contrôle 10 peut comporter un ou plusieurs dispositifs d'entrée tels qu'un clavier, une souris, un stylo, une entrée vocale, etc. et/ou un ou plusieurs dispositifs de sortie tels qu'un ou des écrans, des haut-parleurs, une imprimante, etc. L'environnement peut également comprendre une ou plusieurs connexions de communication, telles que des connexions de type LAN, WAN, point à point, etc. Dans certains modes de réalisation, les connexions peuvent être utilisées pour établir des communications point à point, des communications filaires, des communications sans fil, etc.

Le dispositif de contrôle 10 peut être un ordinateur unique fonctionnant dans un environnement en réseau utilisant des connexions logiques avec un ou plusieurs ordinateurs distants. L'ordinateur distant peut être une station de revue, un ordinateur personnel, un serveur, un routeur, un PC de réseau, un dispositif homologue ou un autre noeud de réseau commun, et peut comprendre généralement plusieurs ou tous les éléments décrits ci-dessus ainsi que d'autres non mentionnés. Les connexions logiques peuvent inclure toute méthode supportée par les supports de communication disponibles.

Comme déjà indiqué, les modes de réalisation du système à ultrasons SY1 sont présentés ci-dessus à titre purement illustratif, d'autres mises en oeuvre étant possible dans le cadre de la présente divulgation.

Par ailleurs, le dispositif de détermination 30 comprend des moyens de traitement pour obtenir et traiter les données de scan (ou données ultrasonores) DT1a et DT1b générées par le système à ultrasons SY1 (figure 3). Les données de scan générées par le système SY1 peuvent être analysées par le dispositif de détermination 30 soit en temps réel, par exemple au moyen d'un algorithme et/ou d'un module d'intelligence artificielle, soit analysées ultérieurement et/ou dans un autre lieu que celui dans lequel se trouve le système à ultrasons SY1.

Comme décrit plus en détails ci-après, la nature des données de scan DT1a et DT1b fournies par le système échographique SY1 au dispositif de détermination 30 peuvent varier selon le cas. Ces données peuvent comprendre par exemple des données d'image (ou données vidéo) et/ou d'autres données obtenues à partir des données de scan ou les données de scan elle mêmes.

Plus spécifiquement, dans l'exemple représenté en figure 3, le dispositif de détermination 30 comprend au moins un processeur 31 et au moins un support lisible par ordinateur tel qu'une mémoire non volatile 32. Cette mémoire 32, réinscriptible ou de type ROM, constitue un support d'enregistrement (ou support d'informations) conforme à un mode particulier de réalisation, lisible par le processeur 31, et sur lequel est enregistré un programme d'ordinateur PG1 conforme à un mode de réalisation particulier. Ce programme d'ordinateur PG1 comporte des instructions pour l'exécution des étapes d'un procédé de détermination selon au moins un mode particulier de réalisation. Ce programme peut notamment définir des instructions pour traiter des données ultrasonores et/ou des instructions pour construire des images illustratives du milieu observé.

Le programme d'ordinateur PG1 peut par exemple comprendre au moins un algorithme d'intelligence artificielle (IA) exécutable par le processeur 31 pour mettre en oeuvre le procédé de détermination selon un mode particulier de réalisation de la présente divulgation. Le programme d'ordinateur PG1 peut en particulier définir un modèle d'intelligence artificielle (ou modèle prédictif) entraîné au moyen de données d'entraînement pour traiter les données ultrasonores obtenues selon le procédé de détermination de la présente divulgation, notamment pour déterminer un ou des indicateurs (ou scores) comme décrit ci-après dans des exemples de réalisation.

Les supports lisibles par ordinateur du dispositif de détermination 30, tels que la mémoire 32, peuvent être n'importe quel support disponible auquel le processeur 31 ou son environnement d'exploitation peuvent accéder. À titre d'exemple, et sans limitation, les supports lisibles par ordinateur peuvent comprendre des supports de stockage informatique et des supports de communication. Les supports de stockage informatique comprennent des supports volatils et non volatils, amovibles et non amovibles, mis en oeuvre dans tout procédé ou technologie de stockage d'informations telles que des instructions lisibles par ordinateur, des structures de données, des modules de programme ou d'autres données. Les supports de stockage informatique ne comprennent pas les supports de communication.

Selon un exemple, le dispositif de détermination 30 est configurée pour recevoir des données de scan, telles que les données DT1a et DT1b, fournies par le système à ultrasons SY1, traiter ces données, et éventuellement envoyer un résultat de ce traitement à un dispositif externe, comme par exemple, un dispositif de stockage, d'affichage, un serveur, ou tout autre dispositif externe.

La communication entre le dispositif de détermination 30 et le système ultrasonore SY1 peut s'effectuer selon une quelconque liaison de communication, de type filaire ou sans fil selon le cas.

La figure 4 représente schématiquement un exemple de réalisation du système à ultrasons SY1 tel que précédemment décrit en référence à la figure 3, à savoir un système d'imagerie ultrasonore dans les exemples considérés.

Le système d'imagerie ultrasonore 10 tel que représenté en figure 4 comprend ainsi :
- le dispositif émetteur 20 prenant la forme d'une sonde à ultrasons,
- une unité de traitement 40 configurée pour traiter ou générer une image sur la base des signaux ultrasonores reçus depuis la sonde 20 (cette unité 40 correspondant par exemple à l'unité de traitement 11 de la figure 3),
- un panneau de commande 42 associé, lié ou connecté à l'unité de traitement 40, ledit panneau de commande pouvant comprendre au moins l'un parmi des boutons 41 et une tablette tactile 42, et
- un ou plusieurs écrans 50 configurés pour afficher la ou les images générées par l'unité de traitement 40.

La sonde 20 peut être connectée à l'unité de traitement 40 par tout moyen de connexion approprié tel qu'un câble 21 ou une connexion sans fil. La sonde 20 est en outre capable d'émettre des ondes ultrasonores W dans un milieu M, et éventuellement aussi de recevoir des ondes ultrasonores W du milieu M, lesdites ondes ultrasonores reçues pouvant alors être conséquentes ou résultant de réflexions desdites ondes ultrasonores émises sur des particules diffusantes à l'intérieur dudit milieu.

L'unité de traitement 40 peut comprendre des dispositifs récepteurs (non représentés), comprenant (ou étant) par exemple des circuits récepteurs, configurés pour traiter (par exemple amplifier et/ou filtrer) des signaux électriques reçus de la sonde 20. Ces dispositifs récepteurs peuvent par exemple comprendre des convertisseurs pour transformer les signaux reçus en données représentant le signal (par exemple des convertisseurs analogiques-numériques (ADC) configurés pour transformer une tension en un code numérique). Les données ultrasonores obtenues peuvent être traitées de diverses manières ; elles peuvent notamment être stockées temporairement dans une mémoire accessible à l'unité de traitement ou traitées directement pour calculer des données traitées intermédiaires (données issues d'un processus de formation de voies, dites « beamformed data » en anglais). L'unité de traitement 30 peut mettre en oeuvre toute méthode de traitement connue pour générer et/ou traiter une ou plusieurs images ou cartes ou données sur la base des signaux reçus de la sonde, telle que la formation de voies.

Les données traitées peuvent être associées à une image ultrasonore de différents natures, lesquelles peuvent être :
- une image B-mode du milieu (image en mode B) généralement affichée en niveaux de gris permettant de visualiser les organes à l'intérieur du milieu, et/ou
- une image dite Doppler, montrant les mouvements de fluides dans le milieu observé, par exemple la vitesse de mouvement et/ou l'écoulement de fluides dans le milieu (image utilisant souvent des codes couleurs), par exemple utile pour visualiser les vaisseaux sanguins et leurs activités dans le milieu, et/ou
- une image montrant une caractéristique mécanique du milieu (élasticité), par exemple utile pour identifier des zones de duretés différentes qui peuvent se révéler être des lésions présentes à l'intérieur du milieu (par exemple des données d'image d'élastographie par ondes de cisaillement (ShearWave^{™} Elastography)).

L'écran d'affichage 50 (par exemple monté sur un bras de support 51) peut être un écran de visualisation de l'image traitée par l'unité de traitement 30 et/ou peut afficher diverses informations selon le cas d'usage, notamment des informations d'aide ou une aide gestuelle contextuelle adaptée ou adaptable au moyen du pavé tactile 42.

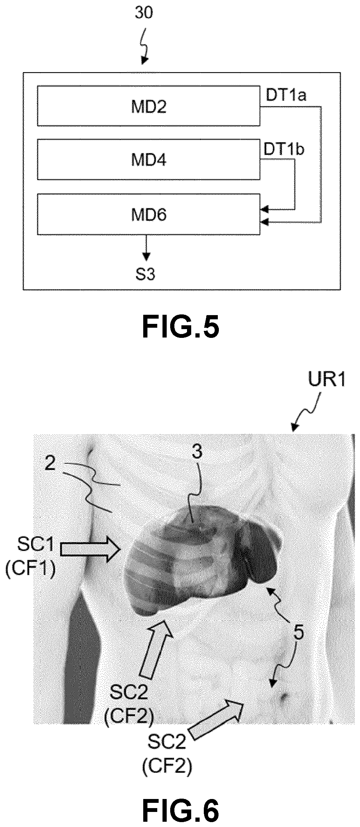

Comme illustré schématiquement en figure 5 selon un exemple, le processeur 31 du procédé de détermination 30, piloté par le programme d'ordinateur PG1 (figure 3), met en oeuvre un certain nombre de modules, à savoir : un premier module d'obtention MD2, un deuxième module d'obtention MD4 et un module de détermination (ou module d'analyse) MD6.

Plus spécifiquement, le premier module d'obtention MD2 est configuré pour obtenir un premier groupe de données de scan DT1a à partir d'ondes ultrasonores W1 se propageant selon une première configuration de scan CF1 du foie 3 (figure 3). Ces premières données de scan DT1a, obtenues au cours d'un premier scan ultrasonores SC1, sont représentatives du foie 3 du sujet UR1.

Le deuxième module d'obtention MD4 est configuré pour obtenir un deuxième groupe de données de scan DT1b à partir d'ondes ultrasonores W1 se propageant selon une deuxième configuration de scan CF2 d'un groupe de tissus du sujet 3 (figure 3). Comme décrit par la suite, ces deuxièmes données de scan DT1a, obtenues au cours d'un deuxième scan ultrasonores SC2, sont représentatives d'un quelconque groupe de tissus, qui peut comprendre tout ou partie du foie 3 du sujet UR1 ou partie (ou région) du sujet UR1 autre que son foie 3 (par exemple un organe de l'abdomen autre que le foie, tel que le pancréas et/ou la vésicule biliaire).

Le module de détermination MD6 est configuré pour déterminer, par une combinaison des premier et deuxième groupes de données de scan DT1a et DT1b, un indicateur (ou indicateur global) S3 représentatif de l'état du foie 3 du sujet UR1. Comme décrit par la suite dans des exemples, le traitement appliqué aux données DT1a et DT1b pour obtenir l'indicateur S3 peuvent être de diverses natures. De même, la nature de l'indicateur S3 peut varier selon le cas.

Des exemples de réalisation de la présente divulgation sont à présent décrits en référence aux figures 6-12. Dans ces exemples, le dispositif de détermination 30 précédemment décrit met en oeuvre le procédé de détermination selon des exemples de réalisation en exécutant le programme PG1 (figure 3). Pour ce faire, le dispositif de détermination 30 coopère avec le système à ultrasons SY1.

On suppose à titre d'exemple que l'on souhaite déterminer l'état du foie 3 d'un sujet UR1 (figures 6-8). Pour ce faire, un praticien, médecin, technicien qualifié, échographiste, ou plus généralement un utilisateur, réalise au moyen du système à ultrasons SY1 (figure 3) deux scans ultrasonores sur le sujet UR1, à savoir :
- un premier scan ultrasonore SC1 au cours duquel des ondes ultrasonores se propagent selon une première configuration de scan CF1 du foie 3 ; et
- un deuxième scan ultrasonore SC2 au cours duquel des ondes ultrasonores se propagent selon une deuxième configuration de scan CF2 de tissus 5 du sujet UR1.

Au cours de chaque scan ultrasonore SC1 et SC2, le système à ultrasons SY1 émet des ondes ultrasonores W1 vers le milieu visé (la partie du sujet UR1 en observation) et reçoit en retour des échos ultrasonores sous la forme d'ondes W2 (figure 3). Ces ondes ultrasonores W2 sont représentatives de la région du sujet UR1 dans laquelle se propagent les ondes émises W1 et les ondes reçues W2. Le système à ultrasons SY1 (et plus particulièrement le dispositif de contrôle 10) génère ainsi des données ultrasonores, dites premières et deuxièmes données de scan DT1a et DT1b, à partir respectivement des échos ultrasonores captées au cours des scans ultrasonores SC1 et SC2.

La ou les régions du sujet UR1 qui font l'objet de l'observation par ultrasons, ainsi que les échos ultrasonores W2 obtenus au cours de cette observation, sont fonction de la configuration des scans ultrasonores SC1 et SC2 réalisés. Divers paramètres peuvent définir ces configurations, dont notamment l'orientation et/ou la position du dispositif émetteur 20 lors des scans ultrasonores.

On suppose à titre d'exemple que le premier scan ultrasonore SC1 est configuré pour scanner (scruter) tout ou partie du foie 3 du sujet UR1. Autrement dit, la configuration de scan CF1 est telle que les ondes ultrasonores se propageant au cours du premier scan SC1 sont représentatives d'une zone d'intérêt (ou région) du foie 3. Le premier scan ultrasonore SC1 est donc un scan d'une ou plusieurs zones d'intérêt du foie.

En outre, on suppose à titre d'exemple que le deuxième scan ultrasonore SC2 est configuré pour viser (ou scruter) un groupe de tissus 5 du sujet UR1 (figure 6), la nature de ces tissus pouvant varier selon le cas. Autrement dit, la configuration de scan CF2 est telle que les ondes ultrasonores se propageant au cours du deuxième scan SC2 sont représentatives du groupe de tissus 5.

Selon un exemple, le groupe de tissus 5 scruté au cours du deuxième scan SC2 comprend tout ou partie du foie (scan du foie). Ainsi, les configuration (ou modes) de scan CF1 et CF2 sont des configurations de scan du foie.

Selon un exemple, le groupe de tissus 5 scruté comprend (ou correspond à) une partie (ou région) du sujet UR1 autre que son foie 3, telle que par exemple tout ou partie de sa vésicule biliaire, d'un organe de l'abdomen et/ou de la graisse viscérale du sujet UR1.

Comme décrit ci-après, la prise en compte d'informations concernant l'état d'une région autre que le foie 3 du sujet peut aider à l'analyse et à la compréhension de l'état du foie 3, notamment car l'état du foie 3 et celui d'autres régions du sujets peuvent être corrélés.

A titre d'exemple, l'observation ultrasonores de la graisse viscérale du sujet UR1 peut apporter des informations utiles pour déterminer l'état du foie 3, notamment en vue d'aider au dépistage ou diagnostic d'un trouble hépatique, car la graisse viscérale peut constituer un biomarqueur d'un état pathologique du foie. Une présence excessive de graisse viscérale peut par exemple être symptomatique de la stéatose hépatique (par exemple de type SHNA), d'une intoxication du foie, ou autre condition du foie.

Le groupe de tissus 5 peut être choisi en fonction d'un type d'examen envisagé, et/ou état supposé du foie que l'on souhaite étudier ou confirmer ou dont on souhaite étudier les évolutions dans le temps ou vis-à-vis d'un traitement, par exemple au cours d'un examen médical. Quelle que soit la région 5 du sujet qui est scrutée au cours du deuxième scan ultrasonore SC2, les première et deuxième configurations de scan CF1 et CF2 sont différentes afin d'obtenir des données ultrasons portant des informations différentes et complémentaires.

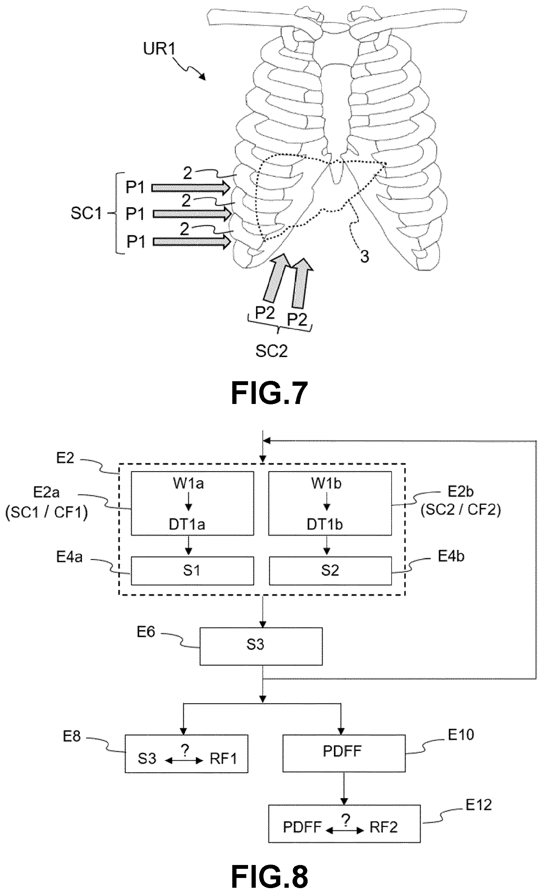

Comme représenté schématiquement en figure 7, on suppose dans ce qui suit à titre purement illustratif que les scans ultrasonores SC1 et SC2 soient configurés de sorte que :
- la première configuration de scan CF1 selon laquelle est réalisé le premier scan ultrasonore SC1 est de type intercostal, c'est-à-dire en faisant propager des ondes ultrasonores au travers (ou entre) les côtes du sujet UR1 (positions P1) ; et
- la deuxième configuration de scan CF2 selon laquelle est réalisé le deuxième scan SC2 est de type sous-costal, c'est-à-dire en faisant propager des ondes ultrasonores sous la cage thoracique au travers de l'abdomen (positions P2).

En positionnant la sonde ultrasonore 20 sur l'abdomen du sujet, il est ainsi possible d'observer le foie du sujet selon une autre coupe, et ainsi améliorer la pertinence des résultats obtenus.

Ainsi, au cours d'une étape E2a (figure 8), le dispositif de détermination 30 obtient un premier groupe de données de scan DT1a à partir d'ondes ultrasonores se propageant au cours du premier scan ultrasonores SC1 selon la première configuration de scan CF1 du foie 3.

De même, au cours d'une étape E2b (figure 8), le dispositif de détermination 30 obtient un deuxième groupe de données de scan DT1b à partir d'ondes ultrasonores se propageant au cours du deuxième scan ultrasonores SC2 selon la deuxième configuration de scan CF2 du groupe de tissus 5.

Pour ce faire, le système à ultrasons SY1 (et plus particulièrement le dispositif de contrôle 10) génère les données de scan DT1a et DT1b à partir des échos ultrasonores reçus au cours respectivement des premier et deuxième scans ultrasonores SC1 et SC2. Ces données de scan DT1a et DT1b sont transmises au dispositif de traitement 30 pour traitement selon le procédé de détermination.

Chaque scan ultrasonores SC1 et SC2 peut comprend un ou une pluralité de scans dans la configuration de scan correspondante CF1 et CF2, respectivement.

Les étapes d'obtention E2a et E2b sont comprises dans une phase d'obtention notée E2 (figure 8). A noter toutefois que divers arrangements des étapes E2a et 2b sont possibles dans le temps. Les étapes E2a et E2b peuvent être réalisées soit simultanément (au moins en partie), soit l'une après l'autre, soit immédiatement après, soit lors d'un autre temps (par exemple lors de séances d'examen différentes). Selon un exemple, le premier groupe de données de scan DT1a est reçu avant le deuxième groupe de données de scan DT1b (ou inversement).

En outre, le dispositif de détermination 30 peut recevoir les données de scan DT1a et DT1b en temps réel, pendant la réalisation des scans SC1 et SC2, ou ultérieurement, de façon groupée ou séparément.

Au cours d'une étape E6 de détermination (figure 8), le dispositif de détermination 30 détermine, par une combinaison des premier et deuxième groupes de données de scan DT1a et DT1b, un indicateur (ou score) S3 représentatif de l'état du foie 3 du sujet UR1. Cette combinaison peut comprendre un traitement prenant en compte les données DT1a et DT1b, la nature de ce traitement pouvant être adaptée selon le cas comme décrit ci-après dans des exemples.

Les étapes E2-E6 peuvent être répétées de façon itératives pour améliorer ou mettre à jour l'indicateur S3 déterminé en E6.

Selon un exemple, l'indicateur S3 définit un (ou au moins un) biomarqueur d'un état physio-pathologique du foie. L'indicateur S3 peut par exemple renseigner sur un trouble hépatique affectant le foie 3 du sujet UR1, par exemple une stéatose hépatique (de type SHNA par exemple), une stéato-hépatite, une intoxication hépatique, etc.

Selon un exemple, l'indicateur S3 déterminé en E6 (figure 8) comprend un coefficient d'atténuation qui représente une atténuation des ondes ultrasonores au cours de leur propagation dans les zones d'intérêt du sujet UR1 lors des premier et deuxième scans ultrasonores SC1 et SC2, respectivement.

Selon un exemple, l'indicateur S3 déterminé en E6 (figure 8) comprend un indicateur définissant par exemple un pourcentage (ou taux) de masse grasse du foie (ou PDFF). Ce pourcentage renseigne sur la quantité moyenne de gras présent dans le foie 3 du sujet UR1.

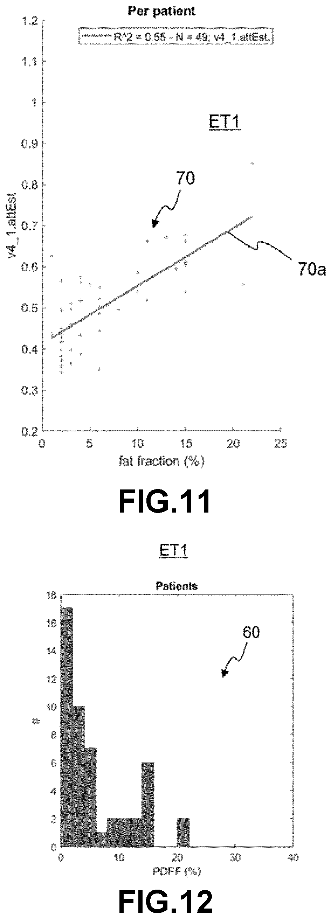

La figure 11 représente, sous forme d'un relevé de points 70, les indicateurs S3 déterminés (E6) pour un groupe de sujets tests au cours d'une étude ET1 réalisée par la déposante. Les indicateurs S3 définissent un taux de masse grasse, ou PDFF, déterminé respectivement pour chaque sujet de l'étude ET1.

La figure 12 représente, à titre de référence, les valeurs de PDFF déterminées pour les sujets identiques de l'étude ET1 par une autre technique que celle du procédé de détermination, à savoir par imagerie IRM. L'évaluation du PDFF par imagerie IRM constitue actuellement la référence pour détecter ou diagnostiquer une stéatose hépatique. A noter que cette étude ET1 concerne en l'espèce des sujets présentant un PDFF inférieur à une valeur limite, à savoir 25%.

L'indicateur S3 déterminé en E6 (figure 8) peut être représentatif d'une quelconque propriété physiologique, mécanique et/ou tissulaire, du foie 3 et/ou ou des tissus 5 scrutés par ultrasons (élasticité, dureté, etc.) au cours des scans ultrasonores SC1 et SC2. L'indicateur S3 peut comprendre un indicateur (ou score) ou une pluralité d'indicateurs (ou scores).

Selon un exemple, au cours du procédé de détermination (figure 8), le dispositif de détermination 30 compare (E8) l'indicateur S3 obtenu en E6 avec une valeur de référence RF1. Cette valeur de référence RF1 peut être une quelconque référence indiquant un état prédéterminé d'un foie (état sain, trouble hépatique particulier, etc.). Par exemple, si le dispositif 30 détecte (E8) que la valeur de l'indicateur S3 obtenue en E6 excède (ou est inférieure à) la valeur de référence, cela signifie que l'état du foie 3 du sujet UR1 est conforme (ou n'est pas conforme) à au moins un critère prédéfini.

Selon un exemple, l'indicateur S3 définit le PDFF du foie 3 du sujet UR1. Au cours de l'étape E8 de comparaison, la valeur de l'indicateur S3 est comparée avec une valeur seuil RF1. Si la valeur obtenue du PDFF excède la valeur seuil RF1(ou se trouve dans une plage de valeurs prédéfinies), alors un état prédéfini du foie 3 est détecté. A titre d'exemple, la valeur du PDFF peut être interprétée comme suit :
- 5,5% pour une stéatose de grade S1 ou supérieur ;
- 15,5% pour une stéatose de grade S2 ou supérieur ; et
- 20,5% pour une stéatose de grade S3 ou supérieur.

Comme indiqué par exemple dans le document: Cassinotto, C., Jacq, T., Anselme, S., Ursic-Bedoya, J., Blanc, P., Faure, S., Belgour, A., & Guiu, B. (2022). Diagnostic Performance of Atténuation to Stage Liver Steatosis with MRI Proton Density Fat Fraction as Reference: A Prospective Comparison of Three US Machines. Radiology, 305(2), 353-361. https://doi.org/10.1148/radiol.212846.

A noter qu'au cours de l'étape E6 (figure 8) de détermination, l'indicateur S3 peut être déterminé de diverses manières en combinant les données de scan DT1a et DT1b résultant des scans ultrasonores SC1 et SC2.

Selon un exemple, les données de scan DT1a et DT1b sont des données qui sont directement combinées par le dispositif de détermination 30 pour en déduire l'indicateur S3. Pour ce faire, le procédé de détermination 30 applique par exemple une fonction de calcul, dite « f1 », sur les données de scan DT1a et DT1b de sorte à produire l'indicateur S3. Autrement dit, on applique la fonction f1 de sorte que : f1(DT1a ; DT1b) = S3

Selon un autre exemple, le dispositif de détermination 30 réalise un premier traitement sur les données de scan DT1a et DT1b avant de déterminer l'indicateur S3. Pour ce faire, le dispositif 30 applique par exemple des fonctions f2 et f3 sur respectivement les données de scan DT1a et DT1b de sorte à produire des scores (ou indicateurs) intermédiaires S1 et S2. Autrement dit, le dispositif de détermination 30 évalue (ou détermine) un premier score S1 (E4a, figure 8) à partir du premier groupe de données de scan DT1a et évalue (ou détermine) un deuxième score S2 (E4b, figure 8) à partir du deuxième groupe de données de scan DT1b. Le dispositif de détermination 30 peut ensuite déterminer (E6) le score S3 à partir des premier et deuxième scores S1 et S2, par exemple en appliquant une fonction f4 sur les scores intermédiaires S1 et S2.

La détermination E6 de l'indicateur S3 revient alors à ce qui suit :
- f2(DT1a) = S1 ;
- f3(DT1b) = S2 ; et
- f4 (S1 ; S2) = S3.

A noter que les types des scores intermédiaires S1 et S2 peuvent varier selon le cas. En particulier, les indicateur S1 et/ou S2 peut chacun comprendre au moins l'un quelconque des types d'indicateur tels que définis ci-après. Par souci de simplification de l'exposé de la présente divulgation, on considère dans les exemples qui suivent que les premier et deuxième indicateurs S1 et S2 comprennent chacun au moins un indicateur tel que défini ci-après dans des exemples particuliers.

Selon un exemple, les indicateurs S1 et S2 déterminés en E4 (figure 8) comprennent chacun un indicateur respectif définissant une vitesse du son, c'est-à-dire la vitesse des ondes ultrasonores se propageant dans le foie du sujet UR1, ou plus précisément dans la ou les zones insonifiées, au cours des scans ultrasonores correspondants SC1 et SC2.

Selon un exemple, les indicateurs S1 et S2 déterminés en E4 (figure 8) comprennent chacun un indicateur respectif définissant un coefficient de rétrodiffusion (dit aussi « backscattering coefficient »), c'est-à-dire un coefficient représentatif d'un niveau de rétrodiffusion causées par la propagation des ondes ultrasonores dans le milieu au cours des scans ultrasonores correspondants SC1 et SC2. Ce coefficient de rétrodiffusion est l'un des paramètres physiques liés à la capacité des milieux sondés à générer un écho se propageant vers la sonde ultrasonore.

Selon un exemple, les indicateurs S1 et S2 déterminés en E4 (figure 8) comprennent chacun un indicateur (ou score) respectif d'élasticité du foie, c'est-à-dire un indicateur représentatif de la capacité des tissus du foie à se déformer en réponse à une contrainte mécanique à laquelle ils sont soumis.

Selon un exemple, les indicateurs S1 et S2 déterminés en E4 (figure 8) comprennent chacun un indicateur (ou score) respectif définissant une viscoélasticité du foie, c'est-à-dire un coefficient représentatif des capacité de viscosité et d'élasticité du foie.

Selon un exemple, les indicateurs S1 et S2 déterminés en E4 (figure 8) comprennent chacun un indicateur (ou score) respectif de non-linéarité de diffusion (ou de non-linéarité de propagation), c'est-à-dire un indicateur représentatif de la non-linéarité de propagation des ondes ultrasonores dans le milieu (dans le foie) au cours des scans ultrasonores SC1 et SC2.

Selon un exemple, les indicateurs S1 et S2 déterminés en E4 (figure 8) comprennent chacun un indicateur (ou score) respectif de diffusion, c'est-à-dire un indicateur représentatif d'un niveau de diffusion causé par la propagation des ondes ultrasonores dans le milieu au cours des scans ultrasonores correspondants SC1 et SC2. Ce coefficient de diffusion peut définir un parcours moyen de diffusion (ou « mean free path » en anglais) qui est fonction de la capacité des tissus insonifiés à générer des échos issus de phénomènes de diffusion multiple.

Il est également possible d'exploiter le phénomène de « speckle » dans une ou des images ultrasonores pour déterminer l'état du foie du sujet. Le speckle ultrasonore (appelé aussi parfois « motif d'interférence » ou « mouchetage ») dans les images ultrasonores est un phénomène de granularité ou de texture apparente qui résulte de la cohérence de l'onde ultrasonore lorsqu'elle interagit avec des structures de tailles variées au sein d'un tissu dans le milieu d'intérêt. Ces motifs granuleux sont le produit de la superposition constructive et destructive des ondes ultrasonores réfléchies par de multiples diffuseurs au sein du tissu, conduisant à une variation aléatoire de l'intensité des signaux reçus. Cette caractéristique peut être utilisée pour extraire des informations représentatives d'un état du foie d'un sujet, telles que la texture des tissus observés, leur homogénéité et/ou d'autres propriétés matérielles.

Ainsi, selon un exemple, les indicateurs S1 et S2 déterminés en E4 (figure 8) comprennent chacun un indicateur (ou score) respectif de distribution de speckle ultrasonore (ou un indicateur de distribution de motif d'interférence). Ces indicateurs (dits aussi indicateurs statistiques de speckle ultrasonore) peuvent caractériser la distribution du phénomène de speckle (ou plus simplement du speckle ou des niveaux de gris) dans une ou des images ultrasonores générées à partir respectivement des premier et deuxième groupes DT1a et DT1b de données de scan (E2, figure 8). Plus particulièrement, à partir des premier et/ou deuxième groupes de données de scan (étapes E2a et E2b), des images ultrasonores B-mode peuvent être générées et, pour chaque image, la distribution des niveaux de gris dans l'image peut être déterminé. Des propriétés statistiques du speckle peuvent ainsi être représentées sous la forme d'au moins un indicateur statistique de speckle, et utilisé le cas échéant pour évaluer l'état du foie du sujet.

Selon un exemple, le premier score S1 est (ou comprend) un premier coefficient d'atténuation et le deuxième score S2 est (ou comprend) un deuxième score d'atténuation. Les scores intermédiaires S1 et S2 sont ainsi représentatifs de l'atténuation des ondes ultrasonores se propageant dans le milieu (les zones d'intérêt) au cours des scans ultrasonores SC1 et SC2, respectivement. Dans ce cas, l'indicateur S3 déterminé en E6 (figure 8) à partir de la combinaison des données de scan DT1a et DT1b (et plus précisément en combinant les scores S1 et S2) peut définir par exemple un biomarqueur d'un état physio-pathologique du foie.

La nature de la fonction f4 utilisée par le dispositif 30 pour déterminer l'indicateur S3 à partir des scores intermédiaires S1 et S2 peut être adaptée selon le cas.

Selon un exemple, l'indicateur S3 est déterminé à partir d'une moyenne des premier et deuxième scores S1 et S2. Cette moyenne est par exemple déterminée par une combinaison linéaire des premier et deuxième scores S1 et S2. Il peut s'agir d'une moyenne simple ou pondérée. Autrement dit, l'indicateur S3 peut être calculé de sorte que S3 = aS1 + bS2, dans lequel les poids a et b sont fixes quelles que soient les valeurs de S1 et S2.

Selon un exemple, la moyenne utilisée pour déterminer S3 est une moyenne linéaire quadratique minimale de S1 et S2 (dit aussi moyenne LSM pour « Least mean-square »).

Selon un exemple, la moyenne de S1 et S2 utilisée pour déterminer (E6, figure 8) l'indicateur S3 est une combinaison non-linéaire des scores S1 et S2. Autrement dit, l'indicateur S3 peut être calculé de sorte que S3 = aS1 + bS2, dans lequel les poids a et b sont variables en fonction des valeurs de S1 et S2. Selon un exemple, l'un des poids a ou b peut être nul pour au moins une valeur du couple (S1, S2), de sorte que seul le score intermédiaire S1 ou S2 ayant un poids non nul est sélectionné et utilisé pour déterminer S3.

Selon un exemple, la combinaison non-linéaire des scores S1 et S2 peut se définir comme f(S1, S2) ou f(DT1a, DT1b), où f désigne une fonction non linéaire.

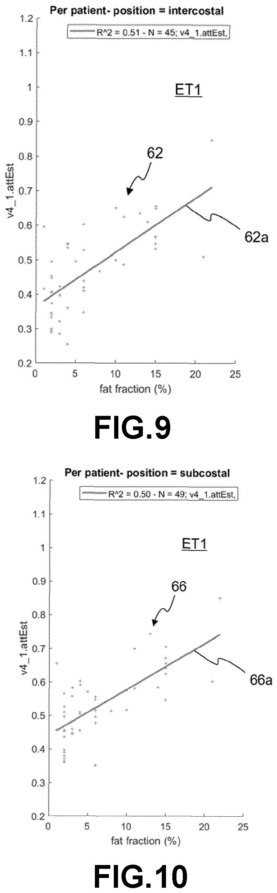

Les figures 9 représente, sous forme d'un relevé de points 62, le score intermédiaire S1 déterminé en E4a (figure 8) à partir des premières données de scan DT1a pour les sujets tests d'une étude notée ET1 réalisée par la déposante. De même, la figures 10 représente, sous forme d'un relevé de points 66, le score intermédiaire S2 déterminé en E4b (figure 8) à partir des deuxièmes données de scan DT1b pour les sujets tests de l'étude ET1. Les scores intermédiaires S1 et S2 définissent dans cet exemples des coefficients d'atténuation caractérisant l'atténuation des ondes ultrasonores se propageant dans le sujet UR1 au cours des premier et deuxième scans ultrasonores SC1 et SC2.

Des régressions linéaires 62a et 66a ont été déterminées à partir respectivement des points 62 et 66 correspondant aux scores S1 et S2 résultant des scans ultrasonores SC1 et SC2 (figures 9 et 10). Ces régressions linéaires 62a et 66a représentent l'évolution générale des coefficients d'atténuation mesurés respectivement en position intercostale et sous-costale en fonction du score PDFF estimé de chaque patient.

Comme cela apparaît à la lecture de l'étude ET1, les performances atteintes dans cet exemple en configuration de scan ultrasonore intercostale et sous-costale sont proches. Les coefficients de détermination, notés respectivement r²_{CF1} et r²_{CF2}, ont été déterminés à partir des valeurs d'atténuation (indicateur S3) obtenues en position (ou mode) intercostal et sous-costal. On obtient ainsi : r²_{CF1} = 0,51 et r²_{CF2} = 0,50. Le coefficient de corrélation traduit la corrélation entre le score PDFF et l'indicateur d'atténuation. Si ce coefficient est égal à 1, alors tous les points sont situés sur une même droite. On obtient ainsi en l'espèce une corrélation légèrement supérieure en configuration sous costale qu'en configuration intercostale.

Pour rappel, le coefficient de détermination r², dit aussi coefficient de détermination linéaire de Pearson, représente la qualité de la prédiction d'une régression linéaire.

La figure 11 représente, sous forme d'un relevé de points 70, les indicateurs S3 déterminés (E6) pour le groupe de sujets tests, évalués au cours de l'étude ET1. Les indicateurs S3 définissent l'indicateur d'atténuation déterminé respectivement pour chaque sujet de l'étude ET1, à partir de la combinaison des coefficients d'atténuation sous costaux et intercostaux de chaque sujet du groupe.

La figure 12 représente, à titre de référence, la distribution des scores PDFF des sujets de l'étude ET1 est déterminée par une autre technique que celle du procédé de détermination, à savoir par imagerie IRM. L'évaluation du PDFF par imagerie IRM constitue actuellement la référence pour détecter ou diagnostiquer une stéatose hépatique. A noter que cette étude ET1 concerne en l'espèce des sujets présentant un PDFF inférieur à une valeur limite, à savoir 25%.

Comme représenté en figure 11, une régression linéaire 70a a également été déterminée à partir des points 70 correspondant aux indicateurs S3 déterminés en E6 (figure 8) pour chaque sujet test de l'étude ET1. Cette régression linéaire 70a représente l'évolution générale du coefficient d'atténuation global en fonction du PDFF des sujets. On constate avantageusement dans cet exemple une amélioration sensible du coefficient de détermination r² qui s'élève à 0,55 pour la régression linéaire 70a. Autrement dit, la régression linéaire 70a présente un meilleur niveau de corrélation des coefficients d'atténuation globaux S3 (que S1 et S2) vis-à-vis du PDFF des sujets, par comparaison avec les résultats obtenus en mode intercostal seul (figure 9) ou en mode sous-costal seul (figure 10). Cela valide pour cet exemple l'efficacité du procédé de détermination qui produit des meilleurs résultats en termes de précision et de fiabilité que si seul un mode d'acquisition (intercostal ou sous-costal) était utilisé.

Selon un exemple illustré en figure 8, une fois l'indicateur S3 déterminé en E6, le dispositif de détermination 30 évalue (E10), à partir l'indicateur S3, un taux de masse grasse, ou PDFF, du foie 3 du sujet UR1. Le dispositif 30 peut éventuellement ensuite comparer (E12) le PDFF obtenu avec une valeur de référence RF2, cette valeur de référence étant par exemple déterminée par une technique d'imagerie IRM ou autre technologie.

Par ailleurs, les premières données de scan DT1a peuvent être obtenues avant, ou en même temps, ou après les deuxièmes données de scan DT1b. En particulier, les premier et deuxième scans ultrasonores SC1 et SC2 peuvent être réalisés simultanément ou l'un après l'autre, selon un ordre quelconque (SC1 avant SC2 ou SC2 avant SC1), durant une même session de scan ou des sessions différentes.

Selon un exemple, l'un parmi les premier et deuxième groupes de données de scan DT1a et DT1b est obtenu à partir d'un scan, dit scan antérieur, réalisé selon une configuration de scan antérieure et l'autre est obtenu à partir d'un scan, dit scan postérieur, réalisé selon une configuration de scan postérieure, le scan antérieur étant réalisé avant le scan postérieur. La configuration de scan antérieure est alors l'une parmi les première et deuxième configuration de scan CF1 et CF2 et la configuration de scan postérieure est l'autre parmi les première et deuxième configuration de scan CF1 et CF2.

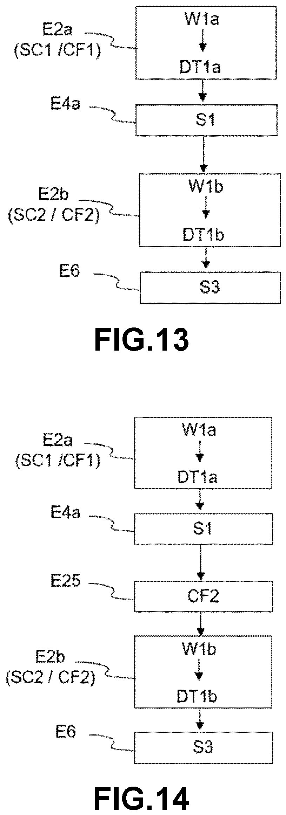

Ainsi, dans un premier exemple représenté en figure 13, le scan antérieur est le premier scan ultrasonore SC1 réalisé selon la configuration de scan antérieure (à savoir CF1) et le scan postérieur est le deuxième scan ultrasonore SC2 réalisé selon la configuration de scan (à savoir CF2). A titre d'exemple, la configuration de scan antérieure est la configuration intercostale et la configuration de scan postérieure est la configuration sous-costale (l'inverse étant possible).

Selon un deuxième exemple, le scan antérieur est le deuxième scan ultrasonore SC2 réalisé selon la configuration de scan antérieure (à savoir CF2) et le scan postérieur est le premier scan ultrasonore SC1 réalisé selon la configuration de scan (à savoir CF1).

Il est ainsi possible de réaliser le scan antérieur, puis ultérieurement le scan postérieur, par exemple au cours d'un même examen médical ou au cours d'examens médicaux distincts dans le temps (par exemple au cours d'un premier examen puis d'un suivi médical). On peut en particulier réaliser le procédé de détermination de la divulgation de façon incrémentale de sorte que le dispositif de détermination 30 génère un premier résultat (par exemple S1 ou S2) à partir du groupe de données de scan résultant du scan antérieur, puis de compléter ce résultat avec les données de scan résultant du scan postérieur afin d'obtenir l'indicateur global S3. De cette manière, on peut avantageusement obtenir rapidement un premier résultat sur l'état du foie 3 du sujet UR1 puis d'améliorer ultérieurement ce résultat grâce au scan postérieur.

Selon un exemple représenté en figure 14, la configuration de scan postérieure du scan postérieur (à savoir la configuration CF2 du scan SC2 dans cet exemple) est sélectionnée (E25) à partir du premier ou deuxième score S1 ou S2 (à savoir S1 dans cet exemple) obtenu à partir du scan antérieur (à savoir SC1 dans cet exemple). Ainsi, on peut avantageusement adapter la manière dont le scan postérieur est réalisé en fonction du résultat précédemment obtenu pour le scan antérieur. En fonction du résultat du scan antérieur, il peut être judicieux de privilégier une configuration de scan particulière pour orienter au mieux l'observation ultrasonore.

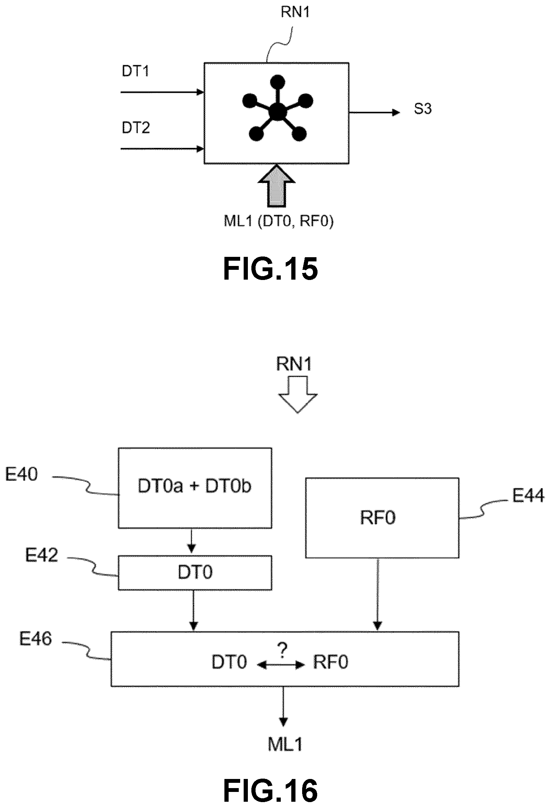

Selon un exemple représenté en figure 15, le dispositif de détermination 30 (figure 3) réalise au cours de l'étape E6 de détermination (figure 8) un traitement des premier et deuxième groupes de données de scan DT1a et DT1b, ou de données obtenues à partir de ces données DT1a et DT1b, pour déterminer l'indicateur global S3. Ce traitement est réalisé selon un modèle de traitement ML1 obtenu en utilisant un (ou au moins un) réseau de neurones RN1 entraîné à partir de données d'entraînement DT0 et de scores (ou indicateurs, ou valeurs) de référence RF0. Pour ce faire, le réseau neuronal RN1 peut exécuter un algorithme basé sur un module d'intelligence artificielle. Les données d'entraînement avec lesquelles est entraîné le réseau de neurones RN1 peuvent comprendre des données de scan ultrasonores ou des données obtenues à partir de telles données de scan ultrasonores.

Selon un exemple représenté en figure 16, préalablement au traitement susmentionné, le réseau de neurones RN1 est entraîné par apprentissage machine au cours d'une phase d'entraînement E40-E46. Au cours de cette phase d'entraînement, le réseau de neurones RN1 obtient (E40) des données de scan ultrasonores DT0a et DT0b représentatives d'ondes se propageant respectivement selon les première et deuxième configurations de scan CF1 et CF2 sur des sujets tests. Le réseau de neurones RN1 détermine (E42) ensuite des données d'entrainement DT0 à partir des données de scan ultrasonores DT0a et DT0b. Ces donnée d'entraînement DT0 peuvent comprennent les données de scan DT0a et/ou DT0b elles-mêmes ou des données obtenues à partir de ces données DT0a et/ou DT0b. Le réseau de neurones RN1 obtient (E44) en outre, à partir d'imageries IRM réalisés sur les sujets tests, des scores de référence RF0 représentatifs de l'état du foie des sujets tests. Le réseau de neurones RN1 peut ainsi déterminer (E46), par comparaison des données d'entraînement DT0 avec les scores de référence RF0, le modèle de traitement ML1 utilisé pour estimer l'indicateur S3 à partir de la combinaison des premier et deuxième groupes de données de scan DT1a et DT1b.

Selon un exemple, au cours de l'étape E6 de détermination (figure 8), le dispositif de détermination 30 exécute successivement un premier algorithme puis un deuxième algorithme pour évaluer l'état du foie 3 du sujet UR1. Le premier algorithme est un algorithme d'estimation configuré pour déterminer un coefficient d'atténuation global S3 à partir des premier et deuxième groupes de données de scan DT1a et DT1b obtenus en mode intercostal et sous-costal, respectivement. Cette première estimation se fait par exemple par moyennage des coefficients d'atténuation S1 et S2 obtenus en inter et sous-costal, comme déjà décrit. Le deuxième algorithme est exécuté par un réseau de neurones (tel que le réseau RN1) pour estimer le PDFF du foie 3 du sujet UR1 à partir du coefficient d'atténuation global S3, par exemple à partir du modèle ML1 ou d'une régression linéaire comme précédemment décrit. Le modèle statistique utilisé dans le deuxième algorithme peut ainsi faire la corrélation entre l'indicateur global S3 (représentant par exemple au moins l'un des paramètres précédemment cités : coefficients d'atténuation, vitesse du son, etc.) et une estimation du PDFF en sortie.

Selon un exemple, le dispositif de détermination 30 peut mettre en oeuvre un réseau de neurones RN1 pour générer un modèle ML1 faisant la corrélation entre des images ultrasonores acquises en entrée à partir des scans ultrasonores SC1 et SC2, d'une part, et l'indicateur S3 ou le PDFF obtenu en sortie.

Le procédé et le dispositif de détermination de la présente divulgation permettent avantageusement de déterminer de façon fiable et performante l'état du foie d'un sujet qui peut être sain ou présenter un état physio-pathologique quelconque du foie. Pour ce faire, le concept de la présente divulgation s'appuie sur la combinaison de données de scan (ou données ultrasonores) obtenues selon deux configurations de scan distinctes, à savoir selon respectivement une première configuration de scan CF1 visant le foie et une deuxième configuration de scan CF2 visant le foie ou un autre groupe de tissus du sujet. La combinaison de ces données de scan permet avantageusement d'obtenir un indicateur S3 représentant de façon plus robuste l'état du foie du sujet que si une seule configuration de scan était utilisée.

La combinaison de deux modes d'acquisition distincts, par exemple en position (ou mode) intercostal et sous-costal, permet avantageusement d'améliorer la qualité des données d'acquisition (avec des données de natures différentes) par rapport à une technique conventionnelle où seules des données ultrasonores obtenues en mode intercostal seraient prises en compte. Les données ultrasonores ainsi acquises sont moins corrélées les unes aux autres. Le recours à deux configurations de scan distinctes permet avantageusement de diversifier les mesures et ainsi augmenter la fiabilité et la précision des résultats.

En outre, dans le cas de la stéatose hépatique par exemple, toutes les régions du foie du sujet peuvent ne pas être affectées de la même manière. Certains cas de stéatoses non uniformes ont ainsi été constatés. La multiplication des scans ultrasonores selon des configurations différentes permet d'obtenir une estimation plus représentative de l'état global du foie du sujet.

La présente divulgation peut ainsi avantageusement de fournir une aide au diagnostic ou à la surveillance de certains états physio-pathologiques du foie, tels que la stéatose hépatiques (par exemple de type SHNA), la stéato-hépatite, etc.

Comme le comprend l'homme du métier, tous les modes de réalisation et variantes décrits ci-avant dont certains ont été simplifiés à dessein pour faciliter les explications, ne constituent que des exemples non limitatifs de mise en oeuvre de la présente divulgation. En particulier, l'homme du métier pourra envisager une quelconque adaptation ou combinaison des modes de réalisation et variantes décrits ci-avant, afin de répondre à un besoin particulier.

La présente divulgation ne se limite donc pas aux exemples de réalisation décrits ci-avant mais s'étend notamment à un procédé de pilotage qui inclurait des étapes secondaires sans pour cela sortir de la portée de la présente divulgation. Il en serait de même d'un système de pilotage pour la mise en oeuvre d'un tel procédé.

## Revendications

1. Procédé de détermination de l'état du foie (3) d'un sujet (UR1), ledit procédé comprenant :
- obtention (E2a) d'un premier groupe de données de scan (DT1a) à partir d'ondes ultrasonores se propageant selon une première configuration de scan (CF1) du foie ;
- obtention (E2b) d'un deuxième groupe de données de scan (DT1b) à partir d'ondes ultrasonores se propageant selon une deuxième configuration de scan (CF2) d'un groupe de tissus (5) du sujet ; et
- détermination (E6), par une combinaison des premier et deuxième groupes de données de scan, d'un indicateur S3 représentatif de l'état du foie (3).

2. Procédé selon la revendication 1, dans lequel :
- le groupe de tissus (5) du sujet correspond à une partie du sujet autre que le foie (3).

3. Procédé selon la revendication 1, dans lequel :
- la première configuration de scan (CF1) est une configuration de scan intercostal du foie ; et
- la deuxième configuration de scan (CF2) est une configuration de scan sous-costal du foie.

4. Procédé selon l'une quelconque des revendications précédentes, comprenant :
- comparaison de l'indicateur S3 avec une valeur de référence (RF1).

5. Procédé selon l'une quelconque des revendications précédentes, dans lequel ledit procédé comprend :
- évaluation (E4a) d'un premier score (S1) à partir du premier groupe de données de scan (DT1a) ; et
- évaluation (E4b) d'un deuxième score (S2) à partir du deuxième groupe de données de scan (DT1b) ;
dans lequel l'indicateur S3 est déterminé à partir des premier et deuxième scores.

6. Procédé selon la revendication 5, dans lequel le premier l'indicateur S1 et/ou le deuxième indicateur S2 comprennent au moins l'un parmi :
- un indicateur définissant un coefficient d'atténuation ;
- un indicateur définissant une vitesse du son ;
- un indicateur définissant un coefficient de rétrodiffusion ;
- un indicateur d'élasticité du foie ;
- un indicateur définissant une viscoélasticité du foie ;
- un indicateur de non-linéarité de propagation ;
- un indicateur de diffusion d'ondes ultrasonores ; et
- un indicateur de distribution de speckle ultrasonore.

7. Procédé selon la revendication 5 ou 6, dans lequel :
- le premier score (S1) est un premier coefficient d'atténuation ; et
- le deuxième score (S2) est un deuxième coefficient d'atténuation ;
dans lequel l'indicateur S3 définit un biomarqueur d'un état physio-pathologique du foie.

8. Procédé selon la revendication 7, dans lequel l'un parmi les premier et deuxième groupes de données de scan (DT1a, DT1b) est obtenu à partir d'un scan dit scan antérieur réalisé selon une configuration de scan antérieure et l'autre est obtenu à partir d'un scan dit scan postérieur réalisé selon une configuration de scan postérieure, le scan antérieur étant réalisé avant le scan postérieur ;
dans lequel la configuration de scan antérieure est l'une parmi les première et deuxième configuration de scan (CF1, CF2) et la configuration de scan postérieure est l'autre parmi les première et deuxième configuration de scan.

9. Procédé selon la revendication 8, dans lequel la configuration de scan postérieure est sélectionnée à partir du premier ou deuxième score (S1, S2) obtenu à partir du scan antérieur.

10. Procédé selon l'une quelconque des revendications 6 à 9, dans lequel l'indicateur S3 est déterminé à partir d'une moyenne des premier et deuxième scores (S1, S2).

11. Procédé selon la revendication 10, dans lequel la moyenne est déterminée par une combinaison linéaire des premier et deuxième scores (S1, S2).

12. Procédé selon la revendication 11, dans lequel la moyenne est une moyenne linéaire quadratique minimale.

13. Procédé selon la revendication 10, dans lequel la moyenne est déterminée par une combinaison non-linéaire des premier et deuxième scores (S1, S2).

14. Procédé selon la revendication 13, dans lequel la détermination de l'indicateur S3 comprend :
- traitement des premier et deuxième groupes de données (DT1a, DT1b), ou de données obtenues à partir des premier et deuxième groupes de données, selon un modèle de traitement obtenu en utilisant un réseau de neurones (RN1) entraîné à partir de données d'entraînement (DT0) et de scores de référence (RF0),
les données d'entraînement comprenant des données de scan ultrasonores ou des données obtenues à partir des données de scan ultrasonores.

15. Procédé selon la revendication 14, dans lequel préalablement au traitement le réseau de neurones (RN1) est entraîné par apprentissage machine au cours d'une phase d'entraînement comprenant :
- obtention (E40) des données de scan ultrasonores représentatives d'ondes se propageant selon les première et deuxième configurations de scan sur des sujets tests ;
- détermination (E42) des données d'entrainement à partir des données de scan ultrasonores ;
- obtention (E44), à partir d'imageries IRM réalisés sur les sujets tests, des scores de référence représentatifs de l'état du foie des sujets tests ; et
- détermination (E46), par comparaison des données d'entraînement avec les scores de référence, du modèle de traitement (ML1) utilisé pour estimer l'indicateur S3 à partir de la combinaison des premier et deuxième groupes de données de scan.

16. Procédé selon l'une quelconque des revendications précédentes, dans lequel le procédé comprend :
- évaluation, à partir de l'indicateur S3, d'un taux de masse grasse du foie du sujet.

17. Programme d'ordinateur (PG1) comportant des instructions pour la mise en oeuvre du procédé selon l'une quelconque des revendications précédentes, lorsque ces instructions sont exécutées par un processeur.

18. Dispositif (30) de détermination de l'état du foie (3) d'un sujet (UR1), ledit dispositif comprenant :
- un premier module d'obtention (MD2) configuré pour obtenir un premier groupe de données de scan à partir d'ondes ultrasonores se propageant selon une première configuration de scan du foie ;
- un deuxième module d'obtention (MD4) configuré pour obtenir un deuxième groupe de données de scan à partir d'ondes ultrasonores se propageant selon une deuxième configuration de scan d'un groupe de tissus du sujet ; et
- un module d'analyse (MD6) configuré pour déterminer, par une combinaison des premier et deuxième groupes de données de scan, un indicateur S3 représentatif de l'état du foie.

19. Système de détermination (SY2) de l'état du foie d'un sujet comprenant :
- le dispositif de détermination (30) selon la revendication 18 ; et
- un système à ultrasons (SY1) couplé au dispositif de détermination.
